# EUROPEAN PATENT SPECIFICATION

(11) **EP 2 313 774 B1**
(45) Date of publication and mention of the grant of the patent: **05.10.2016**
(21) Application number: 09745166.0
(22) Date of filing: 20.08.2009
(51) Int. Cl.: G01N 33/50

(54) **HIGH THROUGHPUT SYSTEM FOR CFU ASSAY BY THE USE OF HIGH RESOLUTION DIGITAL IMAGING, DIFFERENTIAL STAINING AND AUTOMATED LABORATORY SYSTEM**
SYSTEM MIT HOHEM DURCHSATZ FÜR CFU-TESTVERFAHREN UNTER VERWENDUNG HOCHAUFLÖSENDER DIGITALER BILDGEBUNG, DIFFERENTIELLER ANFÄRBUNG UND EINES AUTOMATISIERTEN LABORSYSTEMS
SYSTÈME À HAUT DÉBIT POUR UN DOSAGE D'UNITÉ DE FORMATION DE COLONIE PAR L'UTILISATION D'UNE IMAGERIE NUMÉRIQUE HAUTE RÉSOLUTION, D'UNE COLORATION DIFFÉRENTIELLE ET D'UN SYSTÈME DE LABORATOIRE AUTOMATISÉ

(30) Priority: 20.08.2008 US 90491 P
(43) Date of publication of application: 27.04.2011
(73) Proprietor: New York Blood Center, Inc., New York, NY 10065 (US)
(72) Inventor: ALBANO, Maria, S., New York, NY 10028 (US); ROTHMAN, William, New York, NY 10065 (US); RUBINSTEIN, Pablo, New York, NY 10065 (US)
(74) Representative: Green, Mark Charles
(86) International application number: PCT/US2009/054545
(87) International publication number: WO 2010/022288

(56) References cited:
- EP-A1- 1 865 315
- CROSTA ET AL: "Scoring CFU-GM colonies in vitro by data fusion: A first account" EXPERIMENTAL HEMATOLOGY, NEW YORK, NY, US, vol. 35, no. 1, 30 December 2006 (2006-12-30), pages 1-12, XP005819158 ISSN: 0301-472X
- ALBANO M S ET AL: "Colony-Forming-Unit (CFU) Assay With High-Resolution Digital Imaging: A Reliable System for Cord Blood (CB) CFU Evaluation" BIOLOGY OF BLOOD AND MARROW TRANSPLANTATION, KLUGE CARDEN JENNINGS PUBLISHING, CHARLOTTESVILLE, VA, US, vol. 15, no. 2, 1 February 2009 (2009-02-01), page 45, XP026036262 ISSN: 1083-8791 [retrieved on 2009-02-01]
- ROK BERNARD ET AL: 'Model-based automated detection of mammalian cell colonies' PHYSICS IN MEDICINE AND BIOLOGY vol. 46, no. 11, 01 November 2001, pages 3061 - 3072, XP055002192 DOI: 10.1088/0031-9155/46/11/320 ISSN: 0031-9155
- ANONYMOUS: 'TECHNICAL MANUAL: Mouse Colony - Forming Cell (CFC) Assays Using MethoCult', [Online] 01 June 2005, XP055124912 Retrieved from the Internet: <URL:http://www.stemcell.com/~/media/Techni cal Resources/8/3/E/9/0/28405_methocult M.pdf> [retrieved on 2014-06-24]
- CROSTA ET AL: "Scoring CFU-GM colonies in vitro by data fusion: A first account", EXPERIMENTAL HEMATOLOGY, ELSEVIER INC, US, vol. 35, no. 1, 30 December 2006 (2006-12-30), pages 1-12, XP005819158, ISSN: 0301-472X, DOI: DOI:10.1016/J.EXPHEM.2006.08.015

## Description

### Field of the Invention

The present invention relates to high throughput systems for the objective, standardized determination of colony forming units in populations of hematopoietic cells using a cell detection system and high resolution image analysis.

### Background of the Invention

Umbilical cord blood (CB) is an increasingly accepted graft source for patients lacking related donors. Graft characteristics currently used as determinants of quality and engraftment potential of CB units include the enumeration of total nucleated cells (TNC), CD34+ cells, and colony forming units (CFU). The number of CFU before and after freezing/thawing specimens is a strong independent predictor of CB cell engraftment. Currently, the 14 day CFU assay is the only method that determines the functional state as well as the repopulation capacity and number of hematopoietic progenitor cells. Evaluation of CFU growth as is performed under light microscopy and is time consuming, subjective and difficult to standardize. Further, current methods do not allow for re-counting or visualization of a sample after the assay is complete, as sample plates are discarded at the end of the 14 day assay.

Therefore, a high throughput system to assess CFU number, growth and identify different colony types that is objective and standardized would be desirable. Further, a system that provides positive identification of a sample and storage of an image of the sample and assay results for future reference would be highly desirable.

Crosta GF et al Experimental Hematology 2007, 35, 1 - 12 discloses a methodology for scoring colony forming unit granulocyte macrophage (CFU-GM) colonies *in vitro* by data fusion.

EP 1 865 315 discloses an apparatus including an image-acquisition unit for capturing a plurality of cell images under different image-acquisition conditions along with an analysis unit for extracting a cell position and at least one feature on the basis of the plurality of cell images and for performing statistical analysis with the extracted features serving as parameters.

Bernard R et al Phys Med Biol 2001, 46, 3061 - 3072 discloses a model-based image segmentation method, which employs prior knowledge about the shape of a cell colony with the aim to automatically detect isolated, touching and overlapping cell colonies of various sizes and intensities.

### Summary of the Invention

According to the present invention, there is provided a method for determining the number of hematopoietic precursor cells in a sample comprising:
culturing hematopoietic cells obtained from a sample of a hematopoietic cell-containing tissue or fluid in a semi-solid growth medium layer which supports growth of hematopoietic precursor cell colonies;
staining the colonies in a single step with MTT (3-[4,5-dimethylthiazol-2yl]-2,5-diphenyltetrazolium bromide) without washing;
imaging the colonies in the absence of a microscope; and
counting the colonies' erythroid (erythroid colony forming unit), multilineage (multilineage colony forming unit), and granulocyte-macrophage (granulocyte-macrophage colony forming unit) precursor cells, and combinations thereof, in the sample,
wherein said assay is performed with an image detection and quantification system comprising:
a high resolution camera;
appropriate light alignments;
a broad spectrum flash tube; and
a positive sample identification.

The present disclosure provides a high throughput system for the objective, standardized determination of colony forming units in populations of hematopoietic cells using a cell detection system and high resolution image analysis.

In one embodiment of the disclosure, a standardized colony forming unit assay is provided. The assay comprises the steps of obtaining a sample of a hematopoietic cell-containing tissue or fluid; culturing the cells in a medium that supports growth of hematopoietic precursor cell colonies; staining the colonies; imaging the colonies; counting the colonies for each type of precursor cell; and in certain embodiments, determining the functional state and engraftment potential of the colonies based upon the number of precursor cells and the size of colonies in the sample.

In yet another embodiment, a positive sample identification is provided, comprising a sample and an identification. The sample is a cord blood unit and the identification identifies a specific cord blood unit and information related thereto.

### BRIEF DESCRIPTION OF THE DRAWINGS

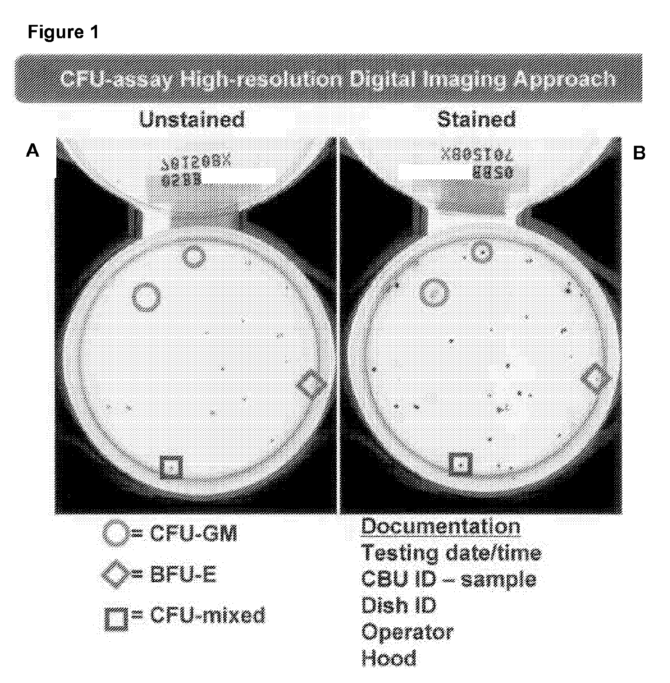

Figure 1 is an image of a 35 mm diameter well comprising unstained (A) and stained (B) CFU (colony forming units) after 14 day cell culture. CFU are identified by the following symbols: o indicates the presence of CFU-GM (granulocyte-macrophage colony forming unit); ◊ indicates the presence of CFU-E (erythroid colony forming unit); and □ indicates the presence of CFU-GM/E (multilineage colony forming unit). Positive identification is provided on the wells, and includes the testing date/time, CBU ID sample, Dish ID, operator identification, and hood information.

### DETAILED DESCRIPTION OF THE INVENTION

The present disclosure provides a high throughput system for the objective, standardized determination of colony forming units (CFU) in populations of hematopoietic cells using colony staining and high resolution digital imaging. A computer-based laboratory information management system supports the high-throughput CFU assay.

In one embodiment of the disclosure, a standardized colony forming unit assay is provided. The assay comprises the steps of obtaining a sample of a hematopoietic cell containing tissue or fluid; culturing the cells in a medium that supports growth of hematopoietic precursor cell colonies; staining the colonies; imaging the colonies; counting the colonies for each type of precursor cells; and in certain embodiments, determining the functional state and engraftment potential of the colonies based upon the number of precursor cells and size of colonies in the sample.

The hematopoietic cells can be obtained from any appropriate source, such as blood (peripheral, umbilical cord blood), bone marrow, placenta, etc. In one embodiment, the source is umbilical cord blood. The cells obtained can be cultured in standard media that supports hematopoietic precursor cell colonies for a period of time, such as about 1-21 days, about 5-14 days, or about 10-14 days. In one embodiment, cells are cultured for 14 days. Examples of suitable media include, but are not limited to AMBION® (Invitrogen), STEMLINE® (Sigma), and STEMSPAN®, ALDEFLUOR®, METHOCULT®, and ALDECOUNT® (Stem Cell Technologies). In one embodiment, it is possible to selectively stimulate the proliferation of discrete cell lineages by using appropriate growth factors. Examples of growth factors appropriate for selection of particular cell lineages include, but are not limited to, erythropoietin for red blood cells, thrombopoietin for megakaryocytes, granulocyte stimulating factor (G-CSF) for granulocytes, and granulocyte macrophage stimulating factor (GM-CSF) for granulocyte and macrophage stimulation. In addition, IL-3, IL-6, and stem cell factor, can also be used. These growth factors can be used alone or in various combinations (i.e., cocktails). The estimation of cell numbers after the incubation of a measured number of cells of a given type in separated or unseparated cell samples provides an alternate method of estimating the proliferative capacity of the corresponding cell lineage in the sample. Thus, the acquisition of colony images from whole culture dishes can be expanded to allow numerical estimation of cell numbers and therefore, of actual cell propagation by comparison with the number of cells seeded. In certain embodiments, a subpopulation of CD34+ or light density enriched cells or apheresis product can be isolated before the culturing step.

The colonies are imaged. Using the disclosed system, CFU can be objectively visualized, differentiated and counted and the digital images can be stored for future review and/or re-classification. The system provides computerized information on optical assay parameters. A combination of high resolution imaging, optional one-step staining, and the traditional CFU assay overcome the technical challenges of the conventional assay. The system supports standardization, classification, and counting reproducibility and high-throughput. The image detection and quantitation system comprises a high resolution camera, appropriate light alignments, a broad spectrum flash tube, and positive sample identification

In one embodiment, a detection system supports the colony forming unit assay. In one embodiment of the detection system, an imaging acquisition system is provided comprising of a full color high speed (for example, 1/125 sec) imaging surface of size comparable to the sample (35mm diameter dish). The imaging system has a ccd detector (for example, about 39 Megapixel or higher) and results in an image resolution on the order of about 7 microns per pixel. This is then coupled with high distortion-free 1:1 macro focusing lens and a custom designed stage that minimizes stray light, provides reproducible positioning between samples and allows for incorporation of human and machine-readable (i.e., bar code) identification imprinted directly onto each image. The excitation consists of an intense uniform broad spectrum flash (about 1/500sec) tube. A vibration-free single image is then acquired by a high performance computer for detailed post-imaging analysis, storage and retrospective studies. In one embodiment, the imaging system is an SLR Camera made by Hasselblad and the light source is by Broncolor. However, any suitable camera and light source can be used. The image detection system allows for the acquisition of an image of the CFU assay culture dish where the CFU grown in the culture can be clearly seen with a clear background. An additional advantage of this system is that it allows for a single sample to be imaged on multiple days in exactly the same physical position due to the alignments provided in the disclosed system. Reproducibly aligning the sample for imaging allows an entire well plate or even a single colony to be visualized repeatedly over a period of time. The growth of the colony or colonies can be tracked over time by comparing the images taken on different days. For example, an image of a sample can be compared on day 1, day 5, and day 14. In this way, the growth of a colony of interest or multiple colonies of interest can be monitored.

The culture is stained. The culture is stained in a single step. The cells are stained in a single step with MTT (3-[4,5-dimethylthiazol-2y1]-2,5-diphenyltetrazolium bromide), without any wash steps, which allows an even better definition of multilineage colony forming units (CFU-GM/E), erythroid colony forming units (CFU-E), and granulocyte-macrophage colony forming units (CFU-GM) by bestowing a specific color on each type (dark purple, red, and light purple respectively) against a uniformly clear background. Specific colors are generated after a specific incubation time and MTT dilution. For instance, in one embodiment, the incubation time can be about 10-45 min. In another embodiment, the incubation time is about 15-40 min. In yet another embodiment, the incubation time is about 30-40 min. The dilution of the dye can vary according to the desired staining. When MTT is used, a dilution of 1/2 to about 1/10 can be used. In one embodiment, a 1/5 dilution of MTT is used. In one embodiment, staining can be achieved with 30 min incubation at 37°C followed by 10 min at room temperature, with a 1/5 dilution of MTT.

The images can be taken before and/or after staining of the culture dishes. In one embodiment the culture is imaged before and after staining. In this embodiment, the cultures can easily be classified and enumerated by comparison of unstained and stained cells and colonies if desired (see Figure 1).

In one embodiment, a computer based laboratory information system (LIMS) is used to monitor and document each step of the CFU assay system. In one embodiment, LIMS provides the storage of culture dish images that are linked by unique barcoded ID labels of a specific CB unit. This provides positive identification of the sample linked to a specific CB unit and stores additional information such as incubator location, plating and counting dates, technician ID, as well as detailed colony enumeration (see Figure 1).

An additional advantage of the LIMS is the portability of the stored information regarding the CB unit. That is, images and data associated with a given sample can be easily sent prior to, or with, shipment of a CB unit that is ordered for a transplant recipient. In this way, the physician of the recipient can view images of the sample and assay, and assess the quality and engraftment potential of the CB sample on site. The stored information can be sent by mail, by fax, and/or electronically. Further portability is achieved when the stored information is sent electronically.

In one embodiment, the number and functional state of the colonies can be compared with and used to estimate the engraftment potential of the cells in the sample can be determined. This is achieved using standard statistical methods standard in clinical outcome analysis, such as the Kaplan Meyer probability estimator (Kaplan, E.L. & Meier, P. (1958). "Nonparametric estimation from incomplete observations", JOURNAL OF THE AMERICAN STATISTICAL ASSOCIATION 53: 457-481) or the cumulative incidence analysis (Gray RJ (1988) A class of K-sample tests for comparing the cumulative incidence of a competing risk, ANNALS OF STATISTICS, 16:1141-1154).

To date, the 14 day CFU assay is the only assay that determines the functional state as well as the number and repopulation capacity of hematopoietic progenitor cells and is routinely performed in laboratories. Evaluation of CFU growth is manually performed by phase contrast light microscopy and CFU colonies have to be classified as CFU-GM/E, CFU-E or CFU-GM based on morphology, color and size and then, manually counted. Thus, the current CFU assay is time consuming, subjective, difficult to standardize and not practical when a large number of samples have to be tested daily. In addition, sample culture dishes cannot be stored and thus, all samples must be analyzed at the end of the culture (day 14) and there is no possibility of review, discussion, re-count and/or re-classification of colonies if needed before the dishes are discarded.

In contrast, the disclosed image detection and quantification system, assay, and methods add the advantage of processing a large number of samples/day. Images can be stored for future review and/or re-classification of colonies. Because of the specific color that each of the colonies acquires after staining, classification is objective and the enumeration of a whole dish can be quickly achieved. More importantly, the new detection system supports positive sample identification, standardization and yields high reproducibility to the assay which can be easily implemented in laboratories where large numbers of samples need to be tested daily.

In addition, the traditional CFU assay needs the use of a light inverted phase microscope to classify and enumerate CFU grown in a dish, which is subjective, and time consuming, and not practical for the enumeration of a large number of culture dishes. By using the presently disclosed system, there is no need for a microscope. Clear definition of different colony types allows a faster colony classification and enumeration and thus, helps to increase productivity. Colonies can be imaged using a variety of spectroscopic techniques, such as but not limited to, absorption and fluorescence. In this way, colony counting and classification can be standardized among different laboratories as it yields higher reproducibility than the conventional classification/enumeration methods. Images can be stored electronically for future review and counting or for physicians. The enumeration of colonies can be automated using colored images. Furthermore, the images can be easily retrieved via a barcoded ID that links them to a specific sample.

A computerized procedure allows the review of the whole CFU assay process (sample loading date and time, sample plating date and time, sample imaging date and time). Technologists who performed each step can be identified by barcoded ID labels on the image ensure positive sample identification and link to a specific sample. Additionally, the disclosed assay, method and system allow the processing of a large number of samples/day, making this a high throughput system. In one embodiment, about 100 dishes can be stained and imaged in about 2 hours. In another embodiment, about 80 dishes can be stained and imaged in less than about 2 hours. In yet another embodiment, about 60 dishes can be stained and imaged in about 70 minutes.

In another embodiment, provided herein is a computerized counting program based on current image analysis methods for identifying objects in the field of view. In one embodiment, colony boundaries are derived and improved color differentiation is achieved. This allows for a more accurate colony count and total area of each type of colony present, which can be correlated to other parameters, such as antigen markers e.g., CD34, CD38, CD79, cell number, donor demographic characteristics, and transplant outcome, drug toxicity, novel growth factors assessment, etc. Further, the assay, method and imaging and detection system provided herein can resolve current problems in experimental counting due to colonies merging or those that have burst into multiple colonies.

In one embodiment, the analysis can be expanded by locating the 2-dimensional limits of a colony in the field of vision, which enables the resolution of distinct colonies growing in close proximity and the calculation of the area occupied by each colony. The definition of the colony border also leads, by addition, to a calculation of the total number of colonies in a dish. When coupled with discriminating stains and/or cell markers defining the cell types in colonies, this approach can provide a 2- or 3-dimensional (area or volume) direct numerical estimate of the proliferation capacity of hematopoietic cells from cord blood and/or other samples, which is of a much higher accuracy than relying solely on the number of colonies. More specifically, in certain embodiments, by obtaining estimates of the third dimension (average depth) using high resolution depth-of-field measurements or by roughly assuming the average depth to be a function of the visible area and the thickness of the semi-solid growth medium layer, volume estimation is permitted. The volume of a colony yields an approximate cell number and by addition allows the estimation of overall cell numbers in all colonies in a dish or just in all colonies of a defined cell type.

Further, depending on the mechanical components utilized in the stage and optical paths, detection and spectroscopic resolution can detect colonies growing at different dimensional levels. In certain embodiments, the computerized alignment of unstained and stained images of an individual culture can be used for a time-dependent comparison between colonies during the 14-day assay procedure.

The photographic images obtained utilizing the disclosed assay and image detection and quantification system permit the computerized storage of assay results as part of a database and are useful for supporting clinical applications, including the release of cord blood units for hematopoietic transplantation.

### Example 1

After 14 days of CB culture (CFU assay, Stem Cell Technologies), an image of a 35 mm dish was captured using a high-resolution photographic camera-based digital imaging system which achieves a resolution of 7.6 µm per pixel and thus allows a clear view of all colonies in the dish with their barcoded identifications. A short one-step staining protocol with MTT (3-[4,5-dimethylthiazol-2y1]-2,5-diphenyltetrazolium bromide) allows definition of CFU-GM/E and CFU-E (dark purple/red) and CFU-GM (light purple) by depicting each cell type in a specific color against a uniformly clear background (see Figure 1). A good correlation was observed after comparison of the new strategy against traditional enumeration by using the microscope (R² linear = 0.95; n=122 culture dishes evaluated). Low variation was observed after 151 cultures were independently classified and enumerated by three different operators (CV (coefficient variation) %=8.9%; range 1-27%) (microscope). Sample plating introduced variation in the CFU assay, in an experiment where nine CB samples were evaluated by multiple plating (intra-assay CV%=21.9%; range 3.4-34.5% and inter-assay CV%=23.3%; range 12.6-35%).

In another embodiment, a computer based laboratory information management system (LIMS) is provided to store the data related to a culture dish which is linked by a unique barcoded identification (ID) label to a specific CB unit. Additional information that can be linked to the CB unit via the barcoded ID includes the CB image, incubator location, plating and counting dates, as well as detailed colony enumeration. This system has been tested on more than 8,000 CB units in duplicate. The specific coloration of CFU colonies allows faster classification and enumeration and thus, permits a more precise analysis of CFU colonies and allows a better determination of their relationship with antigen surface markers like CD34+ cell content, drug toxicity and transplantation engraftment.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Specific embodiments disclosed herein may be further limited in the claims using "consisting of'' or "consisting essentially of'' language. When used in the claims, whether as filed or added per amendment, the transition term "consisting of'' excludes any element, step, or ingredient not specified in the claims. The transition term "consisting essentially of'' limits the scope of a claim to the specified materials or steps and those that do not materially affect the basic and novel characteristic(s).

Furthermore, numerous references have been made to patents and printed publications throughout this specification.

## Claims

1. A method for determining the number of hematopoietic precursor cells in a sample comprising:
culturing hematopoietic cells obtained from a sample of a hematopoietic cell-containing tissue or fluid in a medium which supports growth of hematopoietic precursor cell colonies;
staining the colonies in a single step with MTT (3-[4,5-dimethylthiazol-2yl]-2,5-diphenyltetrazolium bromide) without washing;
imaging the colonies in the absence of a microscope; and
counting the colonies' erythroid (erythroid colony forming unit), multilineage (multilineage colony forming unit), and granulocyte-macrophage (granulocyte-macrophage colony forming unit) precursor cells, and combinations thereof, in the sample,
wherein determining the number of hematopoietic precursor cells is performed with an image detection and quantification system comprising:
a high resolution camera;
appropriate light alignments;
a broad spectrum flash tube; and
a positive sample identification.

2. The method of claim 1, further comprising isolating one or more selected from the group consisting of a subpopulation of CD34+, light density enriched cells, an apheresis product, and a combination thereof, before the culturing step.

3. The method of claim 1, wherein the different precursor cell types are stained different colors.

4. The method of claim 1, further comprising a positive sample identification comprising the sample and an identification, wherein the identification identifies a specific sample and information related thereto.

5. The method of claim 1, wherein the sample is identified with an identification label which identifies a specific sample and information related thereto.

6. The method of claim 1, further comprising the step of imaging the colonies prior to staining.

## Patentansprüche

1. Verfahren zur Bestimmung der Zahl hämatopoetischer Vorläuferzellen in einer Probe, umfassend:
Kultivieren hämatopoetischer Zellen, gewonnen aus einer Probe eines/einer hämatopoetische Zellen enthaltenden Gewebes oder Flüssigkeit in einem Medium, welches das Wachstum hämatopoetischer Vorläuferzellkolonien unterstützt;
Färben der Kolonien in einem einzigen Schritt mit MTT (3-[4,5-Dimethylthiazol-2yl]-2,5-diphenyltetrazoliumbromid) ohne Waschen;
bildliches Darstellen der Kolonien in Abwesenheit eines Mikroskops; und
Zählen der Erythroid- (Erythroid-kolonienbildende Einheit), Multilinien- (Multilinien-kolonienbildende Einheit) und Granulozyten-Makrophagen- (Granulozyten-Makrophagen-kolonienbildende Einheit) Vorläuferzellen der Kolonien, und Kombinationen davon, in der Probe,
wobei die Bestimmung der Zahl hämatopoetischer Vorläuferzellen mit einem bildgebenden Nachweis- und Quantifizierungssystem durchgeführt wird, umfassend:
eine hochauflösende Kamera;
angemessene Ausrichtungen des Lichts;
eine Breitband-Blitzröhre; und
eine positive Probenidentifikation.

2. Verfahren nach Anspruch 1, ferner umfassend das Isolieren von einem oder mehr, ausgewählt aus der Gruppe, bestehend aus einer Subpopulation von CD34+, angereicherten Zellen niedriger Dichte, einem Aphereseprodukt und einer Kombination davon, vor dem Kultivierungsschritt.

3. Verfahren nach Anspruch 1, wobei die verschiedenen Vorläuferzelltypen verschiedenfarbig gefärbt werden.

4. Verfahren nach Anspruch 1, ferner umfassend eine positive Probenidentifikation, umfassend die Probe und eine Identifikation, wobei die Identifikation eine spezifische Probe und darauf bezogene Informationen identifiziert.

5. Verfahren nach Anspruch 1, wobei die Probe mittels eines Identifikationsetiketts identifiziert wird, welches eine spezifische Probe und darauf bezogene Informationen identifiziert.

6. Verfahren nach Anspruch 1, ferner umfassend den Schritt zum bildlichen Darstellen der Kolonien vor dem Färben.

## Revendications

1. Une méthode permettant de déterminer le nombre de cellules précurseurs hématopoïétiques dans un échantillon comprenant :
la culture de cellules hématopoïétiques, obtenues à partir d'un échantillon de tissus ou de fluides contenant des cellules hématopoïétiques, dans un milieu qui permet la croissance des colonies de cellules précurseurs hématopoïétiques ;
la coloration des colonies en une fois avec du MTT (bromure de 3-[4,5-diméthylthiazol-2-yl]-2,5-diphényltétrazolium) sans rinçage ;
l'imagerie des colonies sans microscope ; et
le comptage, pour chaque colonie, des cellules précurseurs érythroïdes (unité formant une colonie érythroïde), multilignées (unité formant une colonie multilignée), et granulocytes-macrophages (unité formant une colonie de granulocytes-macrophages), ainsi que les combinaisons de ces éléments contenus dans l'échantillon,
selon laquelle le nombre de cellules précurseurs hématopoïétiques est déterminé au moyen d'un système de quantification et de détection d'images comprenant :
une caméra haute résolution ;
un réglage adapté de la lumière ;
un tube flash à large spectre ; et
une identification d'échantillon positif.

2. La méthode de la revendication 1, comprenant en outre l'isolement d'un ou plusieurs éléments choisis dans le groupe constitué par la sous-population de cellules enrichies de faible densité CD34+, un produit d'aphérèse et une combinaison de ceux-ci, avant de procéder à l'étape de culture.

3. La méthode de la revendication 1, selon laquelle les différents types de cellules précurseurs sont colorés de différentes couleurs.

4. La méthode de la revendication 1, comprenant en outre une identification d'échantillon positif, incluant l'échantillon et une identification, selon laquelle l'identification permet d'identifier un échantillon précis et les informations lui correspondant.

5. La méthode de la revendication 1, selon laquelle l'échantillon est identifié au moyen d'une étiquette d'identification permettant d'identifier un échantillon précis et les informations lui correspondant.

6. La méthode de la revendication 1, comprenant en outre l'étape d'imagerie des colonies avant leur coloration.
